(19) Europäisches Patentamt
European Patent Office
Office européen des brevets

(11) Veröffentlichungsnummer: **0 543 234 A2**

# (12) EUROPÄISCHE PATENTANMELDUNG

(21) Anmeldenummer: **92119028.6**

(22) Anmeldetag: **06.11.92**

(51) Int. Cl.5: **C07C 68/06**, C07C 69/96, C07C 29/128, C07C 31/20

(30) Priorität: **19.11.91 DE 4137966**

(43) Veröffentlichungstag der Anmeldung: **26.05.93 Patentblatt 93/21**

(84) Benannte Vertragsstaaten: **BE DE ES FR GB IT NL**

(71) Anmelder: **BAYER AG**

**W-5090 Leverkusen 1 Bayerwerk(DE)**

(72) Erfinder: **Mais, Franz-Josef, Dr.**
**Gustav-Poensgen-Strasse 23**
**W-4000 Düsseldorf 1(DE)**
Erfinder: **Buysch, Hans-Josef, Dr.**
**Brandenburger Strasse 28**
**W-4150 Krefeld(DE)**

## (54) Verfahren zur gleichzeitigen Herstellung eines Alkylenglykols und eines Dialkylcarbonats.

(57) Es wird ein Verfahren zur gleichzeitigen Herstellung von Ethylenglykol oder Propylenglykol und Dimethylcarbonat oder Diethylcarbonat durch Umsetzung von Ethylencarbonat bzw. Propylencarbonat mit Methanol oder Ethanol beschrieben. Als Katalysatoren werden hierbei cyclische Amidine der Formel

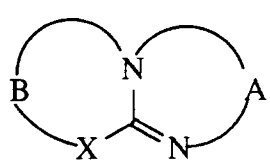

(V)

mit den in der Beschreibung gegebenen Bedeutungen für A, B und X eingesetzt.

EP 0 543 234 A2

Die vorliegende Erfindung betrifft ein Verfahren zur gleichzeitigen Herstellung von Ethylenglykol oder Propylenglykol und Dimethylcarbonat oder Diethylcarbonat durch Umsetzung von Ethylencarbonat bzw. Propylencarbonat mit Methanol oder Ethanol in Gegenwart eines Katalysators.

Ethylenglykol und Propylenglykol sind wertvolle Zwischenprodukte zur Synthese von Polyesterfasern und sind weiterhin wichtige Lösungsmittel. Dimethylcarbonat bzw. Diethylcarbonat sind wertvolle Zwischenprodukte zur Synthese von Polycarbonaten oder Polyurethanen. Die genannten Dialkylcarbonate können weiterhin als Alkylierungsreagenzien, als Lösungsmittel oder als Zusatz zu Treibstoffen für Verbrennungsmotoren Verwendung finden.

Die Umsetzung von Ethylencarbonat mit Methanol oder Ethanol ist bereits bekannt. So wird in US 4.062.884 ein Verfahren vorgeschlagen, in welchem tertiäre Amine als Katalysatoren eingesetzt werden. Das Verfahren besitzt jedoch den Nachteil, daß die genannten Katalysatoren in Mengen von etwa 5 mol-%, bezogen auf das eingesetzte cyclische Carbonat, angewandt werden müssen (Beispiele 1 bis 5 in US'884); es ist weiterhin nachteilig, daß die tertiären Amine die Umsetzung nur sehr langsam katalysieren, was auch durch eigene Nacharbeitung beobachtet werden konnte (Vergleichsbeispiele 1 und 2).

Zur Erhöhung der katalytischen Aktivität der tertiären Amine wurde in JP-A 55/064 550 (1980) vorgeschlagen, als Co-Katalysatoren Lewis-Säuren, wie beispielsweise Zinkchlorid, Eisenchloride, Zinnchloride oder Aluminiumchlorid, zuzusetzen. Dieses Verfahren besitzt jedoch dann wiederum den Nachteil, daß nunmehr eine komplizierte Aufarbeitung der Produktströme zur Abtrennung der anorganischen Verbindungen erforderlich ist. Außerdem können bekanntlich halogenhaltige, besonders chloridhaltige Co-Katalysatoren schon in kleinen Mengen erhebliche Korrosion an den benutzten Apparaturen hervorrufen.

Weiterhin werden in US 4.691.041, EP 298 167, JP-A 63/238 043(1988) und JP-A 03/044 354 (1991) heterogene Systeme als Katalysatoren für die Umsetzung von Ethylencarbonat mit Methanol vorgeschlagen; hierbei handelt es sich um basische oder saure Ionenaustauscher, alkalische, anorganische Feststoffe, beispielsweise $NaSiO_3$ auf Silica oder Hydrotalcit ($MgCO_3 \cdot 5Mg(OH)_2 \cdot 2Al(OH)_3$) oder um Ammonium-ausgetauschte Zeolite, die jedoch ebenfalls nur eine unzureichende katalytische Aktivität besitzen, wie durch eigene Versuche beobachtet wurde (Vergleichsbeispiele 13 und 14).

In EP 150 962 wird ein Verfahren zur Umsetzung von Kohlensäureestern mit Carbonsäureestern in Gegenwart von Amidinen als Katalysatoren beschrieben. Beim Einsatz von Ethylencarbonat und Essigsäureethylester entstehen beispielsweise als Produkte Diethylcarbonat und Ethylenglykoldiacetat. In diesem Verfahren wird zwar die sonst befürchtete Bildung von Nebenprodukten, wie beispielsweise Polyglykolen oder Ethylenglykolmonoethylether vermieden, zur Herstellung des gewünschten Glykols muß aber das anfallende Ethylenglykoldiacetat in einem zusätzlichen Verfahrensschritt zu Ethylenglykol verseift werden.

Es wurde nun ein Verfahren zur gleichzeitigen Herstellung eines Alkylenglykols der Formel

$R^1-CH(OH)-CH_2OH$ (I),

in der $R^1$ für Wasserstoff oder Methyl steht,
und eines Dialkylcarbonats der Formel

$(R^2O)_2CO$ (II),

in der $R^2$ Methyl oder Ethyl bedeutet,
durch Umsetzung eines cyclischen Carbonats der Formel

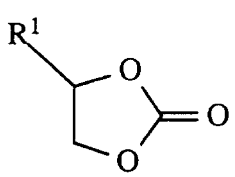

(III)

mit einem Alkohol der Formel

$R^2-OH$ (IV),

worin $R^1$ und $R^2$ die angegebene Bedeutung haben,
in Gegenwart von Katalysatoren gefunden, das dadurch gekennzeichnet ist, daß man als Katalysatoren cyclische Amidine der Formel

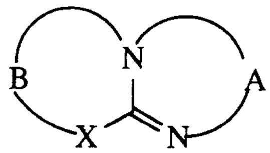

(V)

einsetzt, in denen

A die Gruppe $-(CH_2)_n-$ mit n = 2–4 bedeutet,

B die Gruppe $-(CH_2)_m-$ mit m = 2–5 bedeutet und

X für $CH_2$, O, NH oder $NR^3$ steht, wobei $R^3$ substituiertes oder nicht substituiertes $C_1-C_4$-Alkyl, substituiertes oder nicht substituiertes $C_1-C_4$-Acyl, substituiertes oder nicht substituiertes $C_6-C_{12}$-Aryl oder substituiertes oder nicht substituiertes $C_7-C_{10}$-Aralkyl bedeutet,

und wobei das cyclische Amidin als solches vorliegt oder über eine der Gruppen A, B oder X unter Ersatz eines Wasserstoffatoms in den Gruppen A, B oder X an einen polymeren Träger gebunden ist.

Die erfindungsgemäß einzusetzenden Stoffe sind Ethylencarbonat bzw. Propylencarbonat und Methanol bzw. Ethanol. Die erfindungsgemäß herstellbaren Produkte sind Ethylenglykol bzw. Propylenglykol und Dimethylcarbonat bzw. Diethylcarbonat.

Für den Fall, daß das cyclische Amidin an einen polymeren Träger gebunden ist, seien Träger, wie Polystyrol bzw. Styrol/Divinylbenzol-Copolymer oder Polyacrylsäurederivate bzw. deren vernetzte Vertreter genannt. Als polymerer Träger kommt bevorzugt ein Styrol/Divinylbenzol-Copolymer in Frage. Die Herstellung von an polymere Träger gebundene cyclische Amidine erfolgt in einer dem Fachmann bekannten polymeranalogen Umsetzung.

Bevorzugte cyclische Amidine, wenn diese als solche eingesetzt werden, sind solche aus der Gruppe von

1,5,7-Triaza-bicyclo[4.4.0]-dec-5-en,
1,5-Diaza-bicyclo[4.3.0]-non-5-en,
1,8-Diaza-bicyclo[5.4.0]-undec-7-en,
7-Methyl-1,5,7-triaza-bicyclo[4.4.0]-dec-5-en,
7-Benzyl-1,5,7-triaza-bicyclo[4.4.0]-dec-5-en,
7-Phenyl-1,5,7-triaza-bicyclo[4.4.0)dec-5-en und
7-Acetyl-1,5,7-triaza-bicyclo[4.4.0]-dec-5-en.

Weitere bevorzugte cyclische Amidine für das erfindungsgemäße Verfahren sind solche, die an einen polymeren Träger gebunden sind und durch die folgenden Formeln dargestellt werden:

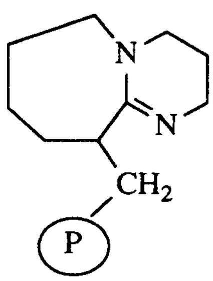

(VI)

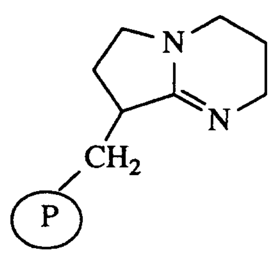

(VII)

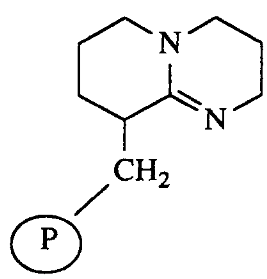

(VIII),

wobei das in den Kreis gestellte Symbol "P" die Kette eines Polymers, beispielsweise die eines Styrol/Divinylbenzol-Copolymers darstellt.

Die Menge an cyclischem Amidin als Katalysator im erfindungsgemäßen Verfahren ist weitgehend unkritisch. Für den Fall, daß die cyclischen Amidine der Formel (V) als solche eingesetzt werden und dann als homogene Katalysatoren vorliegen, wird allgemein eine Menge im Bereich von 0,001–10 mol-%, bevorzugt von 0,01–5 mol-%, bezogen auf das eingesetzte cyclische Carbonat, verwendet. Auch die Menge an cyclischem Amidin, das an einen polymeren Träger gebunden ist, ist weitgehend unkritisch im erfindungsgemäßen Verfahren. Solche polymergebundenen cyclischen Amidine werden in bevorzugter Weise in kontinuierlichen Verfahrensausführungen angewandt. Ihre Einsatzmenge ist daher nicht direkt an die Einsatzmenge der cyclischen Carbonate bzw. der Alkohole gebunden. In bevorzugter Weise werden die

polymergebundenen Katalysatoren in Form einer Säule angeordnet, über die kontinuierlich die Einsatzstoffe entweder im Gleichstrom oder im Gegenstrom geführt werden.

Die Reaktionstemperaturen für das erfindungsgemäße Verfahren liegen im Bereich von 30-250°C, bevorzugt von 50-150°C, besonders bevorzugt von 60-120°C. Der Reaktionsdruck ist beim erfindungsgemäßen Verfahren grundsätzlich unkritisch, so daß bei Normaldruck, Überdruck oder Unterdruck im Bereich von 0,5-100 bar, bevorzugt 1-20 bar, gearbeitet werden kann. In bevorzugter Weise wird bei Normaldruck oder bei dem sich einstellenden Eigendruck bei Reaktionstemperaturen oberhalb des Siedepunktes von mindestens einem der Einsatzstoffe gearbeitet. Es ist grundsätzlich weiterhin möglich, einen Überdruck durch Zugabe von inerten Gasen, wie beispielsweise Stickstoff oder Argon, zu erzeugen, wobei ein Druckbereich von 2-100 bar möglich ist.

Es ist weiterhin möglich, das Reaktionsgemisch durch Zugabe von unter den Reaktionsbedingungen inerten Stoffen zu verdünnen. Dies sind beispielsweise dem Fachmann bekannte Lösungsmittel, wie etwa aliphatische Kohlenwasserstoffe (Hexan, Heptan usw.), aromatische Kohlenwasserstoffe (Benzol, Toluol, Xylol usw.), cyclische Amide (N-Methyl-pyrrolidon, N-Methyl-caprolactam usw.), cyclische Harnstoffe (N,N'-Dimethyl-imidazolidin-2-on usw.). Sofern die als Katalysatoren eingesetzten cyclischen Amidine in solchen inerten Lösungsmitteln löslich sind, können sie zur besseren Dosierung in Form einer 5-50 gew.-%igen, bevorzugt 8-20 gew.-%igen, Lösung in einem solchen Lösungsmittel eingesetzt werden. Es ist jedoch bevorzugt, das erfindungsgemäße Verfahren ohne Mitbenutzung eines solchen inerten Lösungsmittels durchzuführen. Die Variante des Einsatzes cyclischer Amidine in Form einer Lösung der angegebenen Konzentration ist jedoch sehr günstig unter Mitbenutzung der systemeigenen Stoffe Ethylenglykol bzw. Propylenglykol durchführbar; dies stellt demzufolge eine bevorzugte Variante des erfindungsgemäßen Verfahrens dar.

Der Wassergehalt der Ausgangsstoffe und der als Katalysator eingesetzten cyclischen Amidine ist grundsätzlich unkritisch. Zur Unterdrückung unterwünschter Nebenreaktionen sollten die genannten Stoffe jedoch mit dem niedrigen Wassergehalt eingesetzt werden, mit dem sie üblicherweise in der chemischen Technik verfügbar sind. Für den Fall, daß als Alkohol Ethanol eingesetzt wird, ist es bevorzugt, den Wassergehalt in diesem Ethanol unter 1 Gew.-% zu halten. Besonders bevorzugt ist es, den Wassergehalt der Ausgangsstoffe und den des cyclischen Amidins als Summe < 0,5 Gew.-% der genannten Stoffe zu halten.

Das Molverhältnis der eingesetzten cyclischen Carbonate und der eingesetzten Alkohole ist grundsätzlich unkritisch; zur Erzielung eines technisch brauchbaren Ergebnisses ist es jedoch bevorzugt, mindestens 2 mol Alkohol pro 1 mol cyclisches Carbonat einzusetzen; in besonders bevorzugter Weise wird darüber hinaus ein zusätzlicher Überschuß an Alkohol eingesetzt. Das Molverhältnis Alkohol:cyclisches Carbonat beträgt daher im allgemeinen 1-100:1, bevorzugt 2-50:1; besonders bevorzugt 2-12:1.

Das erfindungsgemäße Verfahren kann in verschiedenen Varianten durchgeführt werden. So kann ein Gemisch des cyclischen Carbonats mit dem Alkohol bis zur Einstellung des Gleichgewichts zwischen Einsatzstoffen und Produkten bei erhöhter Temperatur in Gegenwart des Katalysators in einem Rührkessel umgesetzt und nachfolgend durch Destillation getrennt werden.

Ebenso ist es möglich, ein Gemisch der Einsatzstoffe und des Katalysators durch ein erhitztes Rohr, beispielsweise unter Überdruck, zu pumpen und das austretende Produktgemisch in der angegebenen Weise aufzuarbeiten.

Eine weitere Verfahrensvariante besteht darin, daß man aus einem auf Siedetemperatur erhitzten Reaktionsgemisch ein Dampfgemisch aus Alkohol und Dialkylcarbonat abdestilliert.

Weiterhin ist es möglich, in ein über den Siedepunkt des Alkohols erhitztes Gemisch aus dem cyclischen Carbonat und dem cyclischen Amidin den Alkohol als weiteres Ausgangsmaterial dampfförmig einzuleiten und das gebildete Dialkylcarbonat, gegebenenfalls mit überschüssigem Alkohol, als Dampf aus dem Reaktionsraum abzuführen.

Eine noch weitere Verfahrensvariante besteht darin, daß man ein Gemisch der Einsatzstoffe cyclisches Carbonat und cyclisches Amidin bei erhöhter Temperatur im Gegenstrom gegen den Alkohol führt, wobei in vorteilhafter Weise in einer senkrecht stehenden Kolonne gearbeitet wird und wobei am Kopf einer solchen Kolonne das Dialkylcarbonat und der gegebenenfalls überschüssige Alkohol und am Boden der Kolonne das Ethylenglykol bzw. Propylenglykol abgeführt werden. Für den Fall, daß das cyclische Amidin als Katalysator als solches und somit in homogener Phase benutzt wird, fällt es gemeinsam mit dem Ethylenglykol bzw. Propylenglykol am Fuß der Kolonne an.

Es muß als ausgesprochen überraschend angesehen werden, daß man mit sehr niedrigen Katalysatormengen eine sehr rasche Umesterung bei sehr niedrigen Reaktionstemperaturen erreicht. Dies ist technisch außerordentlich günstig, da dann die Bildung an unerwünschten Nebenprodukten sehr gering ist. Die Abtrennung der Katalysatoren ist durch ihren hohen Siedepunkt bei der destillativen Aufarbeitung problem-

los über den Sumpfrückstand möglich. Weiterhin überraschend ist die hohe katalytische Aktivität der erfindungsgemäßen, polymergebunden cyclischen Amidine, die eine wesentlich höhere katalytische Aktivität als die bekannten polymergebundenen Katalysatoren zeigen.

Die folgenden Beispiele verdeutlichen das erfindungsgemäße Verfahren, ohne es jedoch auf diese einzuschränken.

## Beispiele

### Vergleichsbeispiele 1 und 2 und Beispiele 3 – 6

In einem Reaktionsgefäß, ausgestattet mit Rührer, Innenthermometer und Rückflußkühler, wurden 100 Gew. – Tle. Ethanol und die in der Tabelle 1 angegebenen Katalysatoren zum Rückfluß erhitzt. 19,1 Gew. – Tle. Ethylencarbonat wurden auf einmal zugegeben, und die Mischung wurde weiter unter Rückfluß gerührt. Der zeitliche Verlauf der Umsetzung von Ethanol mit Ethylencarbonat zu Ethylenglykol und Diethylcarbonat wurde durch Probennahme und flächengeeichte, gaschromatographische (GC – )Analyse bestimmt. Die Rückflußtemperatur lag während der Umsetzung bei ca. 80 ° C.

Tabelle 1 (Vergleichsbeispiele 1 und 2; Beispiele 3-6)

| Nr. | Katalysator | Katalysatormenge[1] (mol-%) | Rührzeit[2] (h) | Zusammensetzung %[3] EtOH | DEC | EG | EC | Σ Rest |
|---|---|---|---|---|---|---|---|---|
| 1 | $Et_3N$ | 0,5 | 6 | 80,9 | 2,4 | 1,5 | 15,0 | 0,2[4] |
| 2 | $Me_2N-CH_2CH_2OH$ | 0,5 | 6 | 75,4 | 7,9 | 4,6 | 12,0 | 0,1 |
| 3 | 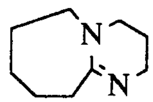 (1,8-Diaza-bicyclo[5.4.0]-undec-7-en) | 0,5 | 2 | 71,8 | 12,3 | 7,0 | 8,9 | 0,02 |
| | | | 4 | 69,5 | 16,5 | 9,1 | 4,9 | 0,02 |
| | | | 6 | 69,3 | 16,7 | 9,5 | 4,5 | 0,02 |
| 4 | 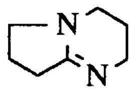 (1,5-Diaza-bicyclo[4.3.0]-non-5-en) | 0,5 | 4 | 70,7 | 14,9 | 8,5 | 5,8 | 0,1 |
| | | | 6 | 69,4 | 17,2 | 9,4 | 3,9 | 0,1 |
| 5 | 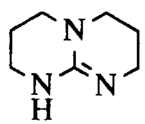 (1,5,7-Triaza-bicyclo-[4.4.0]-dec-5-en) | 0,5 | 1 | 72,6 | 12,6 | 6,9 | 7,9 | 0,02 |
| | | | 2 | 69,9 | 16,9 | 9,1 | 4,1 | 0,01 |
| | | | 4 | 69,7 | 17,3 | 9,3 | 3,7 | 0,02 |
| 6 | 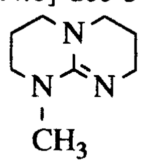 (7-Methyl-1.5.7-triaza-bicyclo[4.4.0]-dec-5-en) | 0,5 | 1 | 72,6 | 13,8 | 7,5 | 6,1 | 0,05 |
| | | | 2 | 68,9 | 17,5 | 9,7 | 3,9 | 0,05 |
| | | | 4 | 68,9 | 17,8 | 9,8 | 3,5 | 0,07 |

Legende zu Tabelle 1:

1) Katalysatormenge, bezogen auf eingesetztes Ethylencarbonat

2) Rührzeit nach Zugabe von Ethylencarbonat

3) GC-Zusammensetzung Einsatzgemisch: 83,9 % EtOH/16,1 % EC (Molverhältnis EtOH/EC = 10:1)

4) Enthält den Katalysator

EtOH: Ethanol
DEC: Diethylcarbonat
EG: Ethylenglykol
EC: Ethylencarbonat.

Vergleichsbeispiel 7 und Beispiele 8 - 11

Das Verfahren des Vergleichsbeispiels 1 und der Beispiele wurde wiederholt, nur daß statt Ethanol nun Methanol als Alkohol und daß 27,5 Gew. - Tle. Ethylencarbonat eingesetzt wurden. Die Rückflußtemperatur lag während der Umsetzung bei 65 - 66 ° C. Die Ergebnisse sind in Tabelle 2 zusammengestellt.

Tabelle 2 (Vergleichsbeispiel 7; Beispiele 8-11)

| Nr. | Katalysator | Katalysatormenge[1] (mol-%) | Rührzeit[2] (h) | Zusammensetzung %[3] EtOH | DMC | EG | EC | Σ Rest |
|---|---|---|---|---|---|---|---|---|
| 7 | $Et_3N$ | 0,5 | 15 | 76,5 | 1,9 | 1,3 | 20,1 | 0,2 [4] |
| | | | 60 | 71,6 | 7,8 | 5,7 | 14,7 | 0,2 |
| 8 | 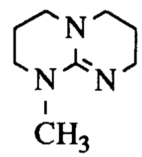 (7-Methyl-1.5.7-triaza-bicyclo[4.4.0]-dec-5-en) | 0,5 | 15 | 63,3 | 19,0 | 13,8 | 3,9 | 0,01 |
| | | | 60 | 63,2 | 19,0 | 14,0 | 3,8 | 0,02 |
| 9 | 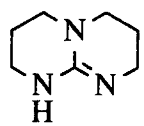 (1.5.7-Triaza-bicyclo-[4.4.0]-dec-5-en) | 0,5 | 15 | 62,8 | 19,0 | 14,3 | 3,9 | 0,04 |
| | | | 60 | 62,7 | 19,0 | 14,4 | 3,9 | 0,04 |
| 10 | 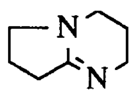 (1,5-Diaza-bicyclo-[4.3.0]-non-5-en) | 0,5 | 15 | 64,8 | 15,8 | 12,0 | 7,4 | 0,07 |
| | | | 60 | 62,8 | 18,9 | 14,0 | 4,3 | 0,07 |
| 11 | 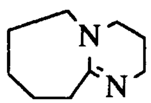 (1,8-Diaza-bicyclo-[5.4.0]-undec-7-en) | 0,5 | 15 | 64,2 | 17,3 | 12,7 | 5,7 | 0,1 |
| | | | 60 | 63,3 | 19,2 | 13,8 | 3,6 | 0,1 |

Legende zu Tabelle 2

1) Katalysatormenge, bezogen auf eingesetztes Ethylencarbonat

2) Rührzeit nach Zugabe von Ethylencarbonat

3) GC-Zusammensetzung Einsatzgemisch: 78,5 % MeOH/21,5 % EC (Molverhältnis MeOH/EC = 10:1)

4) Enthält den Katalysator

MeOH: Methanol
DMC: Dimethylcarbonat
EG: Ethylenglykol
EC: Ethylencarbonat.

Beispiel 12

In einer zehnbödigen, isotherm auf 80 °C beheizten Glockenbodenkolonne von 68 cm Lange und 5 cm Durchmesser (Arbeitsinhalt pro Boden ≈ 11 ml) dosierte man am oberen Ende das Edukt Ethylencarbonat (EC) und auf gleicher Höhe separat eine 13,5 %ige Lösung von 1,8-Diaza-bicyclo[5.4.0]-undec-7-en (DBU) in Glykol ein. Am unteren Ende der Kolonne schickte man diesem ablaufenden Strom Methanol (MeOH) als Dampf entgegen. Am Kopf der Kolonne war über der Dosiereinheit eine unbeheizte verspiegelte 15 cm lange Vigreux-Kolonne als Trenneinheit aufgesetzt. Am unteren Ende befand sich unter der Methanoldampfdosierung ein 30 cm langes Abtriebsteil von 3,5 cm Durchmesser, gefüllt mit 4x4 mm Raschigringen, das auf 120 °C geheizt wurde.

Am Kopf der Vigreux-Trennkolonne wurde der Kopfstrom, enthaltend Dimethylcarbonat (DMC), am unteren Ende des Abtriebsteils der Sumpfstrom, enthaltend Ethylenglykol (EG), entnommen.

Dosierung MeOH: 330 g/h
EC: 100 g/h
13,5 % DBU in Glykol: 13,5 g/h = 1,0 mol-%, bezogen auf EC

| | | |
|---|---|---|
| Abnahme Kopfstrom: | ca. | 354 g/h |
| Sumpfstrom: | ca. | 72 g/h. |

Nach dem Einstellen konstanter Bedingungen (3-4 h) hatten die Ströme folgende Zusammensetzungen:

| | | |
|---|---|---|
| Kopfstrom | MeOH: | 65,7 % |
| | DMC: | 34,2 % |
| | Σ Rest: | 0,1 % |
| Sumpfstrom | MeOH: | 7,8 % |
| | EG: | 91,9 % |
| | Σ Rest: | 0,3 %. |

Vergleichsbeispiele 13 und 14 und Beispiel 15

Das Verfahren der Vergleichsbeispiele 1 und 2 und der Beispiele 3-6 wurde wiederholt, nur daß statt der dort angewandten homogenen löslichen Katalysatoren polymergebundene Katalysatoren eingesetzt

wurden. Die Ergebnisse befinden sich in Tabelle 3.

Tabelle 3 (Vergleichsbeispiele 13 und 14; Beispiel 15)

| Nr. | Katalysator | Katalysatormenge (Gew.-%)[1] | Rühr-zeit (h)[2] | Zusammensetzung %[3] EtOH | DEC | EG | EC | Σ Rest |
|---|---|---|---|---|---|---|---|---|
| 13 | stark basischer Ionentauscher, Polystyrol/5,6 % Divinylbenzol, makroporös Cl-Form, wasserfrei, $-NMe_3^{\oplus}$-Gruppen | 60 | 6 | 80,7 | 2,4 | 1,8 | 14,9 | 0,2 |
| 14 | schwach basischer Ionentauscher/5,6 % Divinylbenzol, makroporös, $-NMe_2$-Gruppen | 60 | 6 | 82,4 | 1,0 | 0,8 | 15,7 | 0,1 |
| 15 | polymergebundenes 1,8-Diaza-bicyclo[5.4.0]-undec-7-en aus Beispiel 16 | 30 | 6 | 76,2 | 6,6 | 4,2 | 12,9 | 0,1 |

1) Menge, bezogen auf Ethylencarbonat
2) Rührzeit nach der Zugabe von Ethylencarbonat
3) GC-Zusammensetzung: Einsatzgemisch 83,9 % EtOH/16,1 %EC (Molverhältnis EtOH/EC = 10:1)

EtOH: Ethanol
DEC: Diethylcarbonat
EG: Ethylenglykol
EC: Ethylencarbonat

Beispiel 16

(Herstellung von polymergebundenem 1,8 – Diaza – bicyclo[5.4.0] – undec – 7 – en)

Bei – 78 °C wurde eine Lösung aus 75 ml abs. Tetrahydrofuran (THF) und 7,6 g 1,8 – Diaza – bicyclo – [5.4.0] – undec – 7 – en gerührt und im Verlaufe von 0,5 h tropfenweise mit einer 1,6 m Lösung von Butyllithium in Hexan versetzt. Anschließend wurde 1 h bei – 78 °C nachgerührt. Dann wurden 5,0 g

chlormethyliertes, getrocknetes Polystyrol (aus Polystyrol mit 4,8 % Divinylbenzol, makroporös, $-CH_2Cl-$ Funktionalisierungsgrad ca. 27 %) zugegeben und das Kältebad entfernt. Bei Raumtemperatur wurde 48 h nachgerührt. Anschließend gab man 20 ml Methanol zu, saugte das Produkt ab und wusch gut mit Methanol, Methanol/$H_2O$ 1:1 und Aceton/Methanol 1:1. Schließlich wurde bei 50°C und 0,1 – 0,2 mbar ca. 6 h getrocknet.

Beispiel 17

Das Verfahren der Beispiele 3 – 6 wurde wiederholt, nur daß statt Ethanol nun Methanol und daß statt Ethylencarbonat nun 31,9 Gew. – Tle. Propylencarbonat sowie 0,5 mol – % 7 – Methyl – 1,5,7 – triaza – bicyclo[4.4.0] – dec – 5 – en, bezogen auf Propylencarbonat, eingesetzt wurden. Die Rückflußtemperatur lag bei ca. 80°C.

15 min nach der Zugabe des Propylencarbonats hatte das Gemisch folgende Zusammensetzung:
65,2 % Methanol
14,8 % Dimethylcarbonat
12,5 % Propylenglykol
7,4 % Propylencarbonat
0,1 % Σ Rest.

Beispiel 18

Das Verfahren der Beispiele 3 – 6 wurde wiederholt, nur daß statt Ethanol nun Methanol und daß 27,5 Gew. – Tle. Ethylencarbonat eingesetzt wurden und daß statt der dort angewandten homogen löslichen Katalysatoren 30 Gew. – Tle. (bezogen auf Ethylencarbonat) des polymergebundenen Katalysators 1,5,7 – Triaza – bicyclo[4.4.0] – dec – 5 – en auf Polystyrol mit 2,0 Gew. – % Divinylbenzol, gelförmig, eingesetzt wurden. Nach 15 min Rührzeit nach Zugabe von Ethylencarbonat unter Rückfluß hatte das Gemisch die folgende Zusammensetzung:
63,5 % Methanol
18,2 % Dimethylcarbonat
14,6 % Ethylenglykol
3,6 % Ethylencarbonat
0,1 % Σ Rest.

**Patentansprüche**

1. Verfahren zur gleichzeitigen Herstellung eines Alkylenglykols der Formel

$R^1-CH(OH)-CH_2OH$ ,

in der $R^1$ für Wasserstoff oder Methyl steht,
und eines Dialkylcarbonats der Formel

$(R^2O)_2CO$ ,

in der $R^2$ Methyl oder Ethyl bedeutet,
durch Umsetzung eines cyclischen Carbonats der Formel

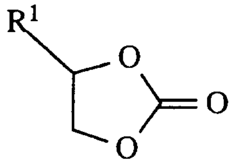

,

mit einem Alkohol der Formel

$R^2-OH$ ,

worin $R^1$ und $R^2$ die angegebene Bedeutung haben, in Gegenwart von Katalysatoren, dadurch gekennzeichnet, daß man als Katalysatoren cyclische Amidine der Formel

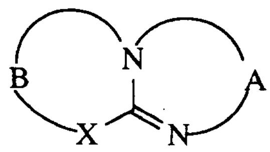

,

einsetzt, in denen

A die Gruppe $-(CH_2)_n-$ mit n = 2–4 bedeutet,

B die Gruppe $-(CH_2)m-$ mit m = 2–5 bedeutet und

X für $CH_2$, O, NH oder $NR^3$ steht, wobei $R^3$ substituiertes oder nicht substituiertes $C_1-C_4$-Alkyl, substituiertes oder nicht substituiertes $C_1-C_4$-Acyl, substituiertes oder nicht substituiertes $C_6-C_{12}$-Aryl oder substituiertes oder nicht substituiertes $C_7-C_{10}$-Aralkyl bedeutet,

und wobei das cyclische Amidin als solches vorliegt oder über eine der Gruppen A, B oder X unter Ersatz eines Wasserstoffatoms in den Gruppen A, B oder X an einen polymeren Träger gebunden ist.

2. Verfahren nach Anspruch 1, dadurch gekennzeichnet, daß man als cyclisches Amidin eines aus der Gruppe von
1,5,7-Triaza-bicyclo[4.4.0]-dec-5-en,
1,5-Diaza-bicyclo[4.3.0]-non-5-en,
1,8-Diaza-bicyclo[5.4.0]-undec-7-en,
7-Methyl-1,5,7-triaza-bicyclo[4.4.0]-dec-5-en,
7-Benzyl-1,5,7-triaza-bicyclo[4.4.0]-dec-5-en,
7-Phenyl-1,5,7-triaza-bicyclo[4.4.0)-dec-5-en und
7-Acetyl-1,5,7-triaza-bicyclo[4.4.0]-dec-5-en,
oder ein an ein Styrol/Divinylbenzol-Copolymer gebundenes cyclisches Amidin aus der Gruppe von

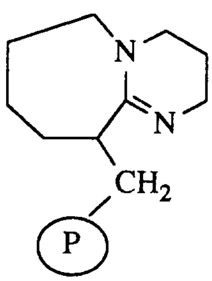

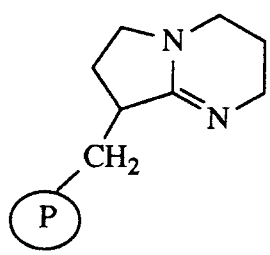

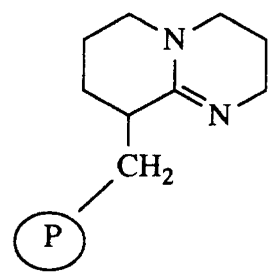

,

wobei das in den Kreis gesetzte Symbol "P" die Kette eines Polymers, bevorzugt die eines Styrol/Divinylbenzol-Copolymers darstellt, einsetzt.

3. Verfahren nach Anspruch 1, dadurch gekennzeichnet, daß man das cyclische Amidin in einer Menge von 0,001–10 mol-%, bevorzugt von 0,01–5 mol-%, bezogen auf das eingesetzte cyclische Carbonat, einsetzt.

4. Verfahren nach Anspruch 1, dadurch gekennzeichnet, daß man bei einer Temperatur von 30–250 °C, bevorzugt von 50–150 °C, besonders bevorzugt von 60–120 °C, arbeitet.

5. Verfahren nach Anspruch 1, dadurch gekennzeichnet, daß man bei einem Gesamtdruck von 0,5–100 bar, bevorzugt von 1–20 bar, arbeitet.

6. Verfahren nach Anspruch 1, dadurch gekennzeichnet, daß man das als solches eingesetzte cyclische Amidin in Form einer Lösung in Ethylenglykol oder Propylenglykol einsetzt.

7. Verfahren nach Anspruch 1, dadurch gekennzeichnet, daß ein Molverhältnis Alkohol:cyclisches Carbonat = 1 – 100:1, bevorzugt 2 – 50:1, besonders bevorzugt 2 – 12:1 eingestellt wird.